# EUROPEAN PATENT SPECIFICATION

(11) **EP 0 471 384 B1**
(45) Date of publication and mention of the grant of the patent: **16.10.1996**
(21) Application number: 91113865.9
(22) Date of filing: 19.08.1991
(51) Int. Cl.: A61F 13/58, A61F 13/15

(54) **Absorbent articles with integral release system and methods of making same**
Absorbierende Gegenstände mit einem integrierten Trennmittelssystem und Verfahren zu deren Herstellung
Articles absorbants avec système intégré de détachement et leur procédé de fabrication

(30) Priority: 17.08.1990 US 568914
(43) Date of publication of application: 19.02.1992
(73) Proprietor: McNEIL-PPC, INC., Milltown New Jersey 08850 (US)
(72) Inventor: Luceri, Thomas Joseph, Nashanic Station, NJ 08853 (US)
(74) Representative: Strehl Schübel-Hopf Groening & Partner

(56) References cited:
- EP-A- 0 182 692
- EP-A- 0 313 426
- DE-U- 7 923 932

## Description

The present invention relates to an absorbent article according to the preamble of claim 1 and to a method of making those absorbent articles. An absorbent article of this type is known from US-A-3 638 651. Those articles may be sanitary napkins, panty liners and the like having integral adhesives for affixing the absorbent article to a user's undergarment.

### BACKGROUND OF THE INVENTION

Absorbent articles such as panty liners and sanitary napkins are well known throughout the art. Generally, these articles have an absorbent, body facing side and a garment facing side. In many instances, the garment facing side is comprised of a fluid impermeable barrier film. A preferred method of securing these articles so that there is a close, direct contact between the perineal area and the user's undergarment is to apply an adhesive to the garment facing side. The adhesive is chosen to provide sufficient anchoring strength to hold the article firmly in place while the user moves.

Inherent in absorbent articles of the above-described construction is the requirement to have release paper applied to the adhesive. The release paper allows the article to be manufactured, packaged, stored, and otherwise handled without the adhesive adhering to itself or any other object. Release paper also serves to prevent oxidation, dust or dirt contamination of the adhesive and evaporation of tackifying resins. Unfortunately, the use of release paper is undesirable for several reasons. First, before the absorbent article can be used, the paper must be removed and discarded. This presents disposal problems and reduces the discretion with which the absorbent articles may be used. Second, release paper adds significantly to product cost. This is particularly true for low cost panty liners and the like which use very little absorbent material. Extra costs due to the release paper are incurred by both the additional material costs and the additional processing time and labor required to apply the release paper.

However, consumers generally desire that such sanitary protection products be stored such that the body-facing layer is protected from the environment and maintained in a clean condition prior to use. Also, consumers prefer to have adhesive applied not only to the center of the pad but to areas as close as possible to the transverse ends of the pad in order to fasten the pad securely in place.

Thus, it would be desirable to eliminate the need for release paper while still retaining the adhesive applied to the garment facing side of an absorbent article so that it can be used to adhere the absorbent article to the undergarment during use but will not stick to itself or an inappropriate surface before use. It would further be desirable to apply such adhesive to the garment facing side of an absorbent article such that a major portion of the body-facing side of the article is protected during storage and such that the adhesive may be applied not only to the central postion of the pad but as close as possible to the transverse ends of the pad.

### SUMMARY OF THE INVENTION

The present invention achieves the above-mentioned improvement by the features mentioned in independant claims 1 and 6. Accordingly, the need for release paper may be eliminated using the method and products of this invention. More particularly, the elimination of release paper may be accomplished by creating release zones and attachment zones at the extreme longitudinal ends of the undergarment-facing side of the products of this invention in such a pattern that the release zones and attachment zones alternate in placement on the undergarment-facing side. Within the attachment zones are located attachment means. Within the release zones are located release means. The attachment and release means are placed such that when the subject absorbent article is folded along a fold line adjacent the longitudinal ends of the undergarment-facing side of the pad, the attachment means meet with the release means.

In accordance with this invention, panty liners are provided which are capable of being stored in a folded configuration prior to use. These absorbent articles have a body-facing side and a garment-facing side. Attachment zones and release zones are created on the garment facing side of the article. Release means are created by depositing a release agent within the release zone by coating at least a portion of the release zone with a release agent. Most preferably, the release agent is silicone, although it may be any of a number of chemical release agents known to those skilled in the art.

Attachment means are created on the garment facing side of the article by delineating attachment zones and depositing attachment or adhesive means to at least a portion of the attachment zones. These attachment zones may be treated to make them more receptive to the attachment means. The attachment means may consist of any means of releasably attaching the absorbent article known to those skilled in the art, for example, pressure-sensitive adhesives, Velcro® hook and loop attachment means or non-slip materials or the like. For the sake of clarity, the following descriptions will exemplify embodiments employing pressure-sensitive adhesive attachment means, however, the description will apply equally to other attachment means.

The patterns in which the adhesive and release means are applied are chosen so that when the article is folded, the attachment means contact the release means, eliminating the need for release paper, but allowing the article to be unfolded and used. The garment facing side of the products of this invention on which the attachment and release zones are located may be polyolefin (e.g., polyethylene or polypropylene) films, non-wovens or the like.

Regardless of the type of attachment means used, the attachment means must bond more strongly to the attachment zone than to the user's undergarment and/or to the release means; the release means must bond more strongly to the release zone than it does to the attachment means and/or to the undergarment; and the attachment means must not bond so strongly to the undergarment that it rips or damages the undergarment by depositing adhesive means on the undergarment.

According to the method of making the products of this invention, there is provided an absorbent article which is folded along a central fold line and two additional fold lines adjacent the longitudinal ends of the product, which remains in the folded position during storage, opens upon demand and which can be attached to the crotch portion of an undergarment. Furthermore, the method and products of this invention permit the wearer to disengage the article from the undergarment without depositing attachment means on the undergarment or tearing the undergarment or the absorbent article.

The ability to accomplish this objective is achieved by controlling the differential "bond strength" with respect to each surface to which the attachment means and the release means are adhered and by placement of the attachment and release means on the undergarment-facing side of the pad. "Peel strength" is a measurement of the strength of an adherent bond. Peel strength is measured in force per unit of width, i.e., the force required to separate adherently joined materials per unit of width. Preferably, a high peel or bond strength should be present between the attachment means and the attachment zones on the garment facing side of the articles of this invention. Likewise, a high peel or bond strength should be present between the release means and the release zones on the garment facing side of the articles of this invention.

A relatively lower peel strength should be present between the release means and the attachment means such that they may be separated prior to use. The differential peel strengths permit the articles of this invention to be stored in a folded position, opened at will without damaging the absorbent article and adhered to an undergarment without damaging the undergarment or absorbent article upon removal.

### BRIEF DESCRIPTION OF THE DRAWINGS

FIG. 1 is a perspective view of a folded panty liner made in accordance with the present invention.

FIG. 2 is a perspective view of the panty liner of Fig. 1 which has been partially opened.

FIG. 3 is a perspective view of the garment-facing side of the panty liner depicted in FIG 1 after having been fully opened.

FIG. 4 is a plan view of the garment-facing side of a panty liner made in accordance with the present invention.

FIG. 5 is a side view of the panty liner of this inventtion.

### DETAILED DESCRIPTION OF THE PREFERRED EMBODIMENTS

The present invention eliminates the need for release paper by providing release means and attachment means in the respective release and attachment zones delineated on the garment-facing side of an absorbent article. The placement of the attachment zones at the extreme longitudinal ends of the undergarment-facing side of the pad permits the symmetrical attachment of the pad in use. Alternating the attachment and release zones transversely across the pad allows placement of attachment means at both extreme ends of the pad, thus providing attachment utility at both ends in the undergarment. The pad will not therefore, disengage itself from the undergarment at either end during use. The folding of the product along selected fold lines in an accordion fashion permits attachment means to be located close to both of the transverse ends of the product while maintaining registry with the release zones when folded.

According to one preferred embodiment, the attachment means are in the form of an adhesive coating. In this embodiment, the release means should have a low enough peel strength with respect to the adhesive coat so as to separate from the adhesive coat without causing damage by stripping adhesive from the attachment zones. The adhesive coat used should have a high enough peel strength with respect to the attachment zone of the garment facing side of the absorbent article to provide sufficient anchorage to prevent adhesive transfer from the article to the undergarment when removing the product from the undergarment after use.

Most preferably, the zones of release and attachment are chosen so that when the product is folded, the adhesive means applied in an attachment zone is covered by the release means within a release zone.

A number of methods may be used to impart the required peel strength characteristics to different zones on the garment facing side of an absorbent article. One method is to use a coating of silicone release agent as the release means applied in the release zones. The attachment zones are left untreated with the silicone coat, and adhesive is applied to at least a portion of the untreated areas. Preferably, the release means are larger in area than the adhesive means such that the adhesive means and release means have a tolerance available for slight folding errors or means placement errors. Preferably, an area of the attachment zones peripherally surrounding the area to which the adhesive is applied may be left uncovered by adhesive. This creates a space around the adhesive such that when the panty liner is folded, there is some tolerance within which the areas of adhesive can avoid contacting the other adhesive means. Furthermore, this provides tolerance for slight errors in adhesive placement within the attachment zone.

Many types of barrier films may be used on the undergarment-facing side of the absorbent to create or allow the creation of attachment and/or release means on that side of the absorbent. For example, the barrier film may be made of polypropylene, high density polyethylene, low density polyethylene, linear low density polyethylene, cellophane, Polyvinyl acetate, polyvinyl alcohol, polycaprolactan, polyester, polytetrafluoroethylene (Teflon®), or mixtures or coextrusions of one or more of these materials. Additionally, films made of materials synthesized to facilitate high moisture vapor transmission could also be used. Highly calendered paper or nonwoven material may also be substituted for films. Further, additives may be combined with the film resin to control the peel or bond strength of the film-to-adhesive bond.

In addition to the above, certain materials, such as Teflon®, produce inherently low adhesive-to-film peel strengths. For these materials, a release coating may not be required in the release zones. Instead, treatment may be necessary to increase the adhesive-film bond in the zones to which adhesive is to be applied. A low-peel strength substrate may be embossed to provide more anchorage surface for the adhesive, or selectively corona treated. The zones to which adhesive will be applied could be either chemically etched or altered to otherwise impart a stronger adhesive-film bond in the attachment zone. In certain embodiments, the adhesive can be applied at a temperature high enough to partially melt the film and produce a "weld" between the film and the adhesive, thereby obviating the need for a separate treatment.

A preferred method of surface treatment is corona treatment of the barrier film which forms the garment facing side of the absorbent article. Corona treatment involves the application of a large voltage across the surface of the film. The resulting treated surface is very reactive and permits the film to form chemical, as well as mechanical, bonds with coatings applied to the surface of the treated article. This provides firmer attachment of both the adhesive and the silicone release coating to the barrier film.

For purposes of securing the absorbent product to the user's undergarment, a wide variety of positioning adhesives are available, pressure sensitive hot melt adhesives being the most widely used and most preferred. These adhesives may be A-B-A block copolymers or the like, such as styrene-ethylene-butylene-styrene block copolymer, e.g., Stereon, or a diblock AB styrene butadiene adhesive. Hot melt adhesives such as HM-6513 or 1972 from H.B. Fuller (St. Paul, Minnesota) or N.S. 34-5509 from National Starch (Bridgewater, New Jersey) are good candidates. Of course, adhesives other than hot melt adhesives may also be used and should be chosen based on the numerous factors such as the compatibility of the adhesives with the other materials being used and the end use of the absorbent product.

The products of this invention may also be made by providing a roll of film which is coated with silicon on one side and adhesive on the other. Strips of this silicone-coated "tape" may be placed on one side of another film which has adhesive on both sides. The tape can be placed in an appropriate pattern (silicone side exposed) against the double adhesive coated film so that the adhesive of one side meets adhesive of the other. This will bond the tape to the film and produce areas of silicone release on the film, i.e., release means. The areas not covered with the tape become the attachment means. Patterns of release and attachment means can be configured such that, when folded, the attachment means is covered by release means.

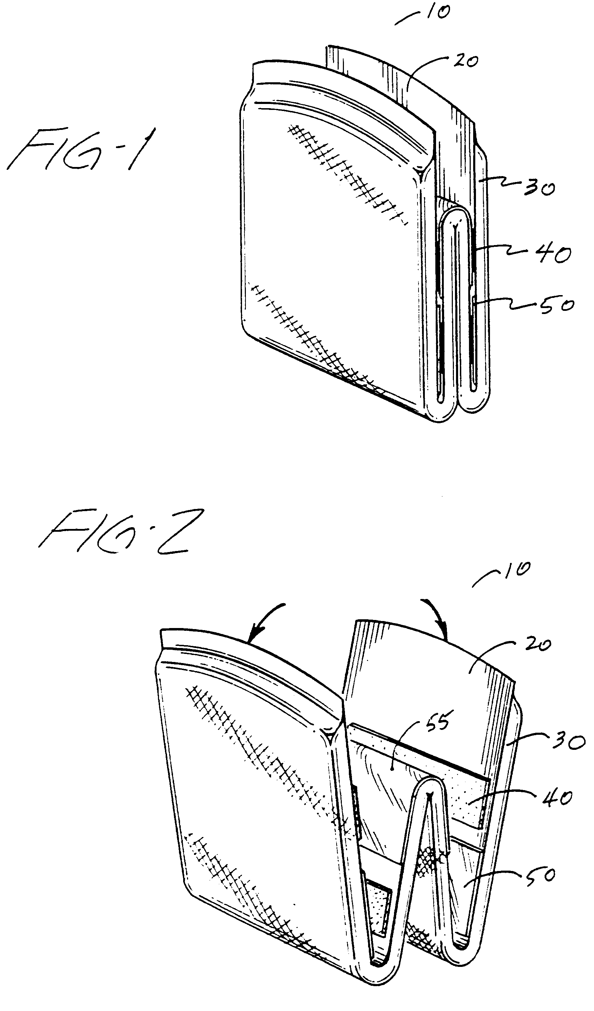

FIGURE 1 illustrates one of the preferred embodiments of the products of this invention. Panty liner 10 has garment-facing side 20 and body-facing side 30. Release means 50 releasably adhere to adhesive means 40. In the folded condition, as depicted in FIGURE 1, panty liner 10 is about one-quarter its fully-opened length and is easy to carry and store.

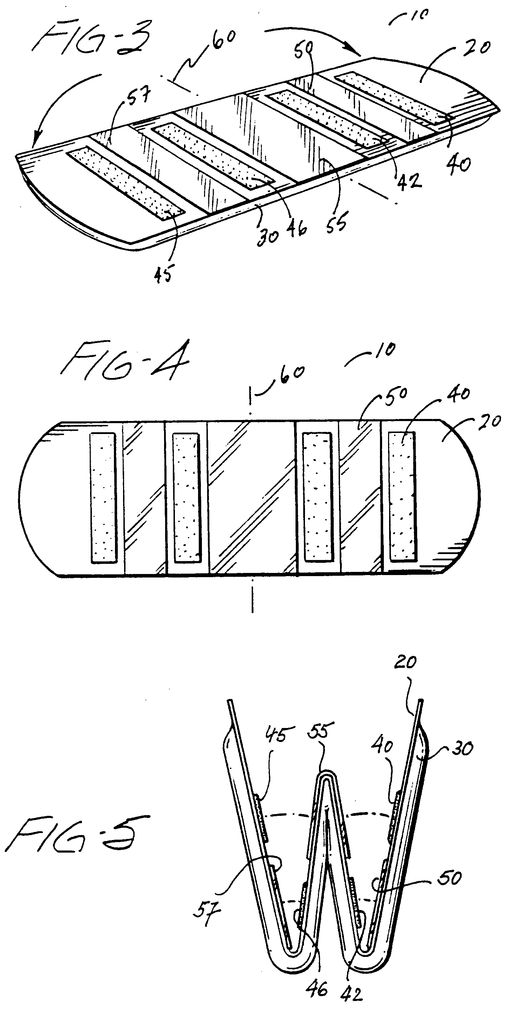

FIGURE 2 depicts the panty liner of FIGURE 1 being partially opened. This illustrates the manner in which adhesive means 40 engages release means 50. Central release means 55 is symmetric about the central axis of panty liner 10 and releasably engages adhesive means 40 and 45 which are disposed at the longitudinal ends of panty liner 10.

FIGURE 3 is a perspective view of panty liner 10 after being fully opened. FIGURE 3 illustrates the symmetry of the release and adhesive patterns about central axis 60 of panty liner 10. Central release means 55 extends symmetrically about central axis 60 and adhesive means 45, 46, 42 and 40 are arranged symmetrically about central axis 60 as well. Release means 52 and 50 are also symmetrically patterned about central axis 60. Release means and adhesive means are arranged such that they are in registry when folded.

Any odd number of folds greater than one may be made symmetrically about the central axis of a pad in order to provide a compact discreet absorbent article according to this invention.

Other embodiments of the products of this invention may be contemplated. For example, an absorbent article may be shaped as a parallelogram with oblique angles at its transverse ends. When folded, the angular ends protrude and become finger tabs that faciliatate unfolding the product.

FIGURE 4 is a plan view of panty liner 10 and FIGURE 5 is a side view of panty liner 10, both illustrating the manner in which the adhesive and release means are arranged symmetrically about the central axis of the pad.

Although one embodiment of the present invention has been illustrated and described in detail, the present invention is in no way so limited. One of ordinary skill will immediately appreciate that the present invention has application to numerous other absorbent articles in addition to the panty liner illustrated.

There are a variety of methods by which the absorbent products of this invention may be made. Preferably, an appropriate barrier film is first printed with a silicone release coating in the release zone. The release coating is cured and adhesive is applied to the attachment zones disposed on the same side as the silicone. Adhesive is also coated on the absorbent facing side of the barrier film to help laminate the film to the absorbent. The adhesive in the attachment zones is preferably applied so as not to occupy the entire surface area of the attachment zones. This provides a tolerance for slight errors in placement of the attachment means in the attachment zone while still substantially guaranteeing the attachment means will lie entirely within the attachment zone. The attachment means is preferably smaller in area than the release means. This allows a tolerance for slight errors in folding the product while substantially guaranteeing the attachment means will be covered by the release means.

The latter side is then affixed to an absorbent substrate, leaving the release coating and adhesive exposed to form the garment facing side of the absorbent article. The finished article is then folded along at least one fold line. The release means and adhesive means contact each other so that the article may be unfolded prior to use without the adhesive means bonding to itself or to the barrier film. The release means may overlap, but the adhesive means must substantially contact only the release means.

For many barriers, treatment is preferred in most cases to impart a low-peel strength surface in the release zones. Silicone-based release agents are excellent for this purpose; these compounds can be applied using many processes other than the screen print/ultraviolet cure set forth below in the Example. In addition to silicon-based release agents, other types of release coats may be used including paraffin waxes, non-stick coatings, varnishes and others known to those of skill in the art. Some types of adhesives become non-tacky when dried or cooled (such as non-pressure sensitive hot melt adhesives or cold glues) and may make suitable release means if used as a coating in the release zone.

Various silicone curing methods may be used, including in addition to ultraviolet curing, heat curing or electron beam curing. The release coating may be applied using a wide variety of coating equipment. Direct and reverse gravure coaters, three-roll offset coaters, smooth five-roll coaters, or ink jet printing are just a few of the possible equipment types contemplated for use in the present invention.

The following Example further illustrates the preferred embodiments of the present invention:

### EXAMPLE

A panty liner according to this invention was made as follows. A roll of barrier film of 38.1 µm (1.5 mil) white microembossed polyethylene film Type # EMB533 available from Exxon Corporation, was first slit to about a 155.6 mm (6.125") width. The film was then corona treated to have a surface energy of about 0.0038 N/cm (38 dynes/cm) on the side which was to be coated. Silicone was then printed on the corona-treated side in the configuration shown in FIGURE 1. The silicone was printed using a screen printer manufactured by Kraemer Koating, Inc. (Serial No. 1036, Toms River, New Jersey).

An ultraviolet (UV) curable type silicone release agent which is made by the General Electric Corporation was used. The formula is as follows:

| Percent | Product# | Description |
|---|---|---|
| 98% | UV9300* | UV-curable silicone |
| 2% | UV9310C^{*} | UV-activated catalyst |

| | | |
|---|---|---|
| ^{*}General Electric Product Designations | | |

The silicone release agent was added behind a doctor blade inside the printing screen. The variable speed pump manufactured by Ismatec (Model No. 7617-60, made in Switzerland by ISMATEC SA, Zurich, Switzerland) was used to add the release agent, although any low volume metering pump may be used. The printing screen used is made by New England Rotary Screen, Inc. (Fall River, Massachusetts). A 125 mesh Pantene screen was found to produce an even coating. The coat weight of the silicone was about 3.4 grams/meter²(g/m²). It should be noted, however, that the added coating weight can be reduced greatly with more carefully designed equipment. Ideally, only enough silicone should be applied to impart consistent release characteristics to the coated area.

The silicone release material was then cured using the Mini-Conveyorized UV Curing System from American Ultraviolet Company (Murray Hill, New Jersey). A lamp setting of 118.11 W/cm (300 watts/inch) provided adequate curing over a wide range of speeds, up to almost 121.92 m/min (400 feet/minute). The optimum machine speed using the materials and equipment of this Example is about 60.96 m/min (200 feet/min). The curing step fixed the silicone in the release zone thereby preventing transfer of silicone to the adhesive means.

After the silicone was printed and cured onto the barrier film, adhesive was applied. The adhesive was applied within the attachment zones. Preferably the adhesive is not applied to the entire area of the attachment zones. Applying adhesive means onto a smaller area than the whole of the attachment zone, makes registration of the adhesive means easier within the attachment zone. Generally, creating release means which is larger than the adhesive means area is preferred, because this allows for folding error tolerance.

In this Example, the adhesive was applied after the silicone coating, on a separate adhesive coating apparatus. Yet, both the silicone release coating and adhesive could be applied in sequence on the same machine. The adhesive used in this example was HM-1972 hot melt adhesive from H.B. Fuller Company (St. Paul, Minnesota). The adhesive was applied at a coating weight of about 11.0 mg/cm² (71 mg/inch²) in four zones. Lower coating weights may also be used as long as there is an amount of adhesive sufficient to hold the product in place during use. For the application of this Example, these adhesive strips were about 9.53 mm (three-eighth inches) wide and about 40.64 mm (1.6 inches) long. There was a space of about 38.1 mm (1.5 inches) between the two innermost adhesive strips while there was a 22.225 mm (0.875 inch) space between the outermost adhesive strips. The adhesive was applied to release paper and transferred to the film, the release paper being left in place to aid in rewinding the coated film. The head of the adhesive nozzle was fashioned to produce all adhesive areas at once. The head was alternately turned on and off to provide enough space between the sets of adhesive areas to fashion individual products.

In order to affix the barrier film to an absorbent core or substrate, the same adhesive was sprayed onto the absorbent-facing side of the barrier film to be used as a laminating adhesive. The adhesive may also be extruded onto the absorbent-facing side of the barrier film. Release papers were used to cover the laminating adhesive as well to aid winding the barrier film into rolls. The release papers were discarded when the product was assembled. Release papers were needed here only by virtue of the manual process herein described. No processing release paper would be required if all the steps of the process are performed on the same machine.

The laminating adhesive was then exposed by removing the release paper and an absorbent batt was placed against the laminating adhesive. For this Example, the absorbent core or substrate was a co-form of pulp and thermoplastic fiber substantially similar to that used in the CAREFREE PANTY SHIELDS® brand of panty liners manufactured by Personal Products Company of Milltown, NJ. The absorbent was fashioned as a 107.95 mm (4.25") width material C-folded around a 45.72 mm (1.8 inch) insert of the same material. The final width dimension of the absorbent was approximately 50.8 mm (2.0"). The composite of barrier and absorbent was then crimped on the ends using a knurled block which was hot pressed into the product ends. The product ends were then cut to provide a rounded tab end.

The remaining release paper which covered the adhesive applied to the garment facing side was then removed and the article folded in half along the central fold-line aligned transversely across the napkin such that the body-facing sides come into contact with each other. The transverse ends were folded again such that the release and attachment means meet. The adhesive means contact the spots of release coating, allowing the product to be easily unfolded without damage or the need to discard a release paper.

The present invention is not limited to the Example and embodiments set forth above. As will be understood by those of ordinary skill in the art, alternate embodiments, variations and modifications of the present invention are possible.

Accordingly, reference should be made to the appended claims to ascertain the full scope of the present invention.

## Claims

1. Absorbent article (10) having
- a body-facing side (30) comprising an absorbent body, a garment-facing side (20) comprising a fluid impermeable barrier film means, transverse ends and longitudinal edges; said barrier film means being provided with at least two attachment means comprising pressure sensitive adhesive means (40, 42, 45, 46) and with at least one release means (50, 55, 57),
- one central fold line extending approximately transversely across said absorbent article (10) and being disposed approximately in a central axis (60) between the first and second ends of said absorbent article (10);
said absorbent article (10) being folded along said central fold line (60) such that the adhesive means (40, 42, 45, 46) contacts the release means (50, 55, 57);
said release means (50, 55, 57) being releasably attached to the attachment means;
said adhesive means (40, 42, 45, 46) are completely covered by said release means (50, 55, 57);
characterized by
- said absorbent article being a panty liner (10);
- said adhesive means (40, 42, 45, 46) being applied to the undergarment-facing side (20) by delineating attachment zones and said adhesive means (40, 42, 45, 46) being deposited to at least a portion of the attachment zones to secure said panty liner (10) in close direct contact between the perineal area and the user's undergarment;
- a first (55) of said release means (50, 55, 57) being centrally extended symmetrically about the central axis (60) of the panty liner (10) and a second (50, 57) of said release means (50, 55, 57) being additionally arranged symmetrically about the central axis (60);
- two additional fold lines made symmetrically about the central axis (60) in order to provide a compact discreet panty liner (10);
said central release means (55) releasably engages outermost adhesive means (40 and 45) which are disposed at the longitudinal ends of panty liner (10);
said centrally extended release means (50, 57) releasably engage innermost adhesive means (42, 46);
said release means (50, 55, 57) being larger in area than the adhesive means (40, 42, 45, 46) such that the adhesive means (40, 42, 45, 46) and release means (50, 55, 57) have a tolerance available for slight folding errors or means placement errors eliminating the need for release paper, but allowing the panty liner (10) to be unfolded and used.

2. Absorbent article of claim 1, characterized by
- said adhesive means being adhesive strips (40, 42, 45, 46);
- a space being provided between the two innermost adhesive strips (42, 46) and a space being provided between the outermost adhesive strips (40, 45);
- the composite of barrier and absorbent being crimped on the ends which are being cut to provide a tab end;
- the transverse ends being folded again such that the attachment and the release means (40, 42, 45, 46; 50, 55, 57) meet;
- the panty liner (10) being folded in half along the central fold line (60) such that the body-facing side (30) come into contact with each other;
- the transverse ends of the panty liner (10) being folded again such that the release and attachment means meet, so that the adhesive means (40, 42, 45, 46) contact the spots (50, 55, 57) of release coating, allowing the panty liner (10) to be easily unfolded without damage.

3. The absorbent article of claims 1 or 2, characterized by
- a barrier film of 38.1 micron (1.5 mil) having a width of about 155.6 mm (6.125");
- said barrier film being corona treated to have a surface energy of about 0.0038 N/cm (38 dynes/cm) on the side to be coated;
- an ultraviolet (UV) curable type silicone release agent being printed on the corona-treated side of the barrier film and cured;
- said adhesive strips (40, 42, 45, 46) having a width of about 9.53 mm (3/8 inches) and a length of about 40.64 mm (1.6 inches) applied onto a smaller area than the whole of the attachment zone to create said release means (50, 55, 57) being larger than the adhesive strips (40, 42, 45, 46);
- said space between the two innermost adhesive strips (42, 46) being about 38.1 mm (1.5 inches);
- said space between the outermost adhesive strips (40, 45) being about 22.225 mm (0.875 inch).

4. Absorbent article of claim 1 or 2, characterized by the release agent (50, 55, 57) being silicone.

5. Absorbent article of claim 1 characterized in that the panty liner (10) has the shape of a rectangle.

6. Method of manufacturing an absorbent article (10) as claimed in any of claims 1 to 5, characterized by
- providing a barrier film;
- said barrier film being first printed with a release coating (50, 55, 57) in a release zone;
- adhesive (40, 42, 45, 46) being applied to attachment zones disposed on the same side (20) as the release coating (50, 55, 57);
- adhesive being also coated on an absorbent-facing side (30) of the barrier film to help laminate the film to an absorbent;
- the adhesive (40, 42, 45, 46) in the attachment zones being applied so as not to occupy the entire surface area of the attachment zones;
- the absorbent-facing side (30) of the barrier being affixed to an absorbent substrate, leaving the release coating (50, 55, 57) and adhesive (40, 42, 45, 46) exposed to form the undergarment-facing side (20) of a panty liner (10);
- said release means (50, 55, 57) being releasably attached to the attachment means;
- said panty liner (10) being folded along a central fold line extending approximately transversely across said panty liner (10) and being disposed approximately in a central axis (60) between first and second ends of said panty liner (10);
- said panty liner (10) being folded along two additional fold lines made symmetrically about the central axis (60) in order to provide a compact discreet panty liner (10) remaining in the folded position during storage;
said central release means (55) releasably engages utermost adhesive means (40 and 45) which are disposed at the longitudinal ends of panty liner 10);
- said centrally extended release means (50,57) releasably engage said innermost adhesive means (42,46);
- said release means (50, 55, 57) being larger in area than the adhesive means (40, 42, 45, 46) such that the adhesive means (40, 42, 45, 46) and release means (50, 55, 57) have a tolerance available for slight folding errors or means placement errors eliminating the need for release paper, but allowing the panty liner (10) to be unfolded and used;
- the release means (50. 55, 57) and adhesive means (40, 42, 45, 46) contacting each other so that the panty liner (10) can be unfolded prior to use without the adhesive means (40, 42, 45, 46) bonding to itself or to the barrier film and can be attached to the crotch portion of an undergarment.

7. Method of claim 6, characterized by
- a roll of 38.1 micron (1.5 mil) microembossed polyethylene barrier film being first slit to a 155.6 mm (6.125") width and then corona treated to have a surface energy of about 0.0038 N/cm on the side to be coated;
- silicone being printed on the corona-treated side by using a screen printer;
- an ultraviolet curable type silicone release agent being added behind a doctor blade inside a printing screen;
- curing the silicone release material by using UV curing system and a machine speed of about 60.96 m/min;
- applying hot melt adhesive strips (40, 42, 45, 46) in each of four zones onto a smaller area than the whole of each of the attachment zones;
- spraying the same hot melt adhesive onto the absorbent-facing side of the barrier film to be used as a laminating adhesive and placing the absorbent batt against the laminating adhesive;
- crimping the composite of barrier and absorbent on the ends;
- cutting the product ends to provide a rounded tab end;
- folding the panty liner (10) in half along the central fold line (60) aligned transversely across the panty liner (10) such that the body-facing side (30) come into contact with each other;
- folding the transverse ends again such that the release and attachment means (50, 55, 57) meet, so that the adhesive means (40, 42, 45, 46) contact the spots (50, 55, 57) of release coating, allowing the panty liner (10) to be easily unfolded without damage or the need to discard a release paper.

## Patentansprüche

1. Absorbierender Gegenstand (10) mit
- einer körperzugewandten Seite (30), die einen absorbierenden Körper umfaßt, einer der Kleidung zugewandten Seite (20), die eine flüssigkeitsundurchlässige Sperrfilmeinrichtung umfaßt, quergerichteten Enden und Längskanten; wobei die Sperrfilmeinrichtung mit mindestens zwei Befestigungseinrichtungen, die eine druckempfindliche Hafteinrichtung (40, 42, 45, 46) umfassen, und mit mindestens einer Trenneinrichtung (50, 55, 57) versehen ist;
- einer zentralen Faltlinie, die sich in etwa in Querrichtung zum absorbierenden Gegenstand (10) erstreckt und in etwa in einer zentralen Achse (60) zwischen dem ersten und zweiten Ende des absorbierenden Gegenstands (10) angeordnet ist;
wobei der absorbierende Gegenstand (10) entlang der zentralen Faltlinie (60) so gefaltet ist, daß die Hafteinrichtung (40, 42, 45, 46) die Trenneinrichtung (50, 55, 57) kontaktiert;
wobei die Trenneinrichtung (50, 55, 57) in trennbarer Weise an der Befestigungseinrichtung befestigt ist;
wobei die Hafteinrichtung (40, 42, 45, 46) vollständig von der Trenneinrichtung (50, 55, 57) bedeckt ist;
dadurch gekennzeichnet, daß
- es sich beim absorbierenden Gegenstand um eine Slipeinlage (10) handelt;
- die Hafteinrichtung (40, 42, 45, 46) auf die der Unterwäsche zugewandten Seite (20) durch Skizzieren von Befestigungszonen und durch Abscheiden der Befestigungseinrichtung (40, 42, 45, 46) auf mindestens einen Teil der Befestigungszonen aufgebracht wird, um die Slipeinlage (10) in engem direktem Kontakt zwischen dem perinealem Bereich und der Unterwäsche des Benutzers zu befestigen;
- wobei eine erste Trenneinrichtung (55) der Trenneinrichtungen (50, 55, 57) sich in zentraler Richtung symmetrisch um die zentrale Achse (60) der Slipeinlage (10) erstreckt und eine zweite Trenneinrichtung (50, 57) der Trenneinrichtungen (50, 55, 57) zusätzlich symmetrisch zur zentralen Achse (60) angeordnet ist;
- wobei zwei zusätzliche Faltlinien symmetrisch zur zentralen Achse (60) angebracht sind, um eine kompakte unauffällige Slipeinlage (10) bereitzustellen;
- wobei die zentrale Trenneinrichtung (55) in trennbarer Weise in Eingriff mit den äußersten Hafteinrichtungen (40 und 45) steht, die an den Längsenden der Slipeinlage (10) angeordnet sind;
wobei die vom Zentrum entfernten Trenneinrichtungen (50, 57) in trennbarer Weise im Eingriff mit den innersten Hafteinrichtungen (42, 46) stehen;
wobei die Trenneinrichtungen (50, 55, 57) eine größere Fläche als die Hafteinrichtungen (40, 42, 45, 46) aufweisen, so daß die Hafteinrichtungen (40, 42, 45, 46) und die Trenneinrichtungen (50, 55, 57) einen Spielraum zum Ausgleich bei geringfügigen Faltungsfehlern oder Plazierungsfehlern der Einrichtungen aufweisen, wodurch die Notwendigkeit für Trennpapier entfällt, jedoch die Slipeinlage (10) aufgefaltet und angewandt werden kann.

2. Absorbierender Gegenstand nach Anspruch 1, dadurch gekennzeichnet,
- daß es sich bei den Hafteinrichtungen um Klebestreifen (40, 42, 45, 46) handelt;
- daß ein Zwischenraum zwischen den beiden innersten Klebestreifen (42, 46) und ein Zwischenraum zwischen den beiden äußersten Klebestreifen (40, 45) vorgesehen ist;
- daß der Verbundstoff aus Sperre und absorbierendem Mittel an den Enden, die zur Bereitstellung eines lappenförmigen Endes zugeschnitten sind, gefältelt ist;
- die Querenden erneut so gefaltet sind, daß die Befestigungseinrichtungen und die Trenneinrichtungen (40, 42, 45, 46; 50, 55, 57) sich treffen;
- daß die Slipeinlage (10) in der Mitte entlang ihrer zentralen Faltlinie (60) so gefaltet ist, daß die körperzugewandten Seiten miteinander in Kontakt kommen; und
- die Querenden der Slipeinlage (10) erneut so gefaltet sind, daß die Trenn- und Befestigungseinrichtungen aufeinandertreffen, so daß die Klebeeinrichtungen (40, 42, 45, 46) in Kontakt mit den Stellen (50, 55, 57) der Trennbeschichtung kommen, was eine leichte beschädigungsfreie Auffaltung der Slipeinlage (10) ermöglicht.

3. Absorbierender Gegenstand nach Anspruch 1 oder 2, dadurch gekennzeichnet,
- daß ein 38,1 µm (1,5 mil) dicker Sperrfilm mit einer Breite von etwa 155,6 mm (6,125") vorliegt;
- daß der Sperrfilm einer Koronabehandlung unterzogen worden ist, um eine Oberflächenenergie von etwa 0,0038 N/cm (38 dyn/cm) auf der zu beschichtenden Seite zu gewährleisten;
- daß ein UV-härtbares Silikon-Trennmittel auf die koronabehandelte Seite des Sperrfilms aufgedruckt und gehärtet ist;
- die Klebestreifen (40, 42, 45, 46) mit einer Breite von etwa 9,53 mm (3/8 Zoll) und einer Länge von etwa 40,64 mm (1,6 Zoll) auf eine im Vergleich zur Gesamtheit der Befestigungszone kleinere Fläche aufgebracht ist, um zu gewährleisten, daß die Trenneinrichtungen (50, 55, 57) größer als die Klebestreifen (40, 42, 45, 46) sind;
- daß der Zwischenraum zwischen den beiden innersten Klebestreifen (42, 46) etwa 38,1 mm (1,5 Zoll) beträgt; und
- daß der Zwischenraum zwischen den äußersten Klebestreifen (40, 45) etwa 22,225 mm (0,875 Zoll) beträgt.

4. Absorbierender Gegenstand nach Anspruch 1 oder 2, dadurch gekennzeichnet, daß es sich beim Trennmittel (50, 55, 57) um Silikon handelt.

5. Absorbierender Gegenstand nach Anspruch 1, dadurch gekennzeichnet, daß die Slipeinlage (10) die Form eines Rechtecks aufweist.

6. Verfahren zur Herstellung eines absorbierenden Gegenstands (10) nach einem der Ansprüche 1 bis 5, dadurch gekennzeichnet,
- daß man einen Sperrfilm bereitstellt;
- daß der Sperrfilm zunächst in einer Trennzone mit einer Trennbeschichtung (50, 55, 57) bedruckt wird;
- daß Klebstoff (40, 42, 45, 46) auf die auf der gleichen Seite (20) wie die Trennbeschichtung (50, 55, 57) angeordneten Befestigungszonen aufgebracht ist;
- daß Klebstoff auch auf eine dem Absorptionsmittel zugewandte Seite (30) des Sperrfilms aufgebracht wird, um eine Laminierung des Films auf ein Absorptionsmittel zu unterstützen;
- der Klebstoff (40, 42, 45, 46) in den Befestigungszonen so aufgebracht wird, daß er nicht die gesamte Oberfläche der Befestigungszonen besetzt;
- daß die dem Absorptionsmittel zugewandte Seite (30) der Sperre an einem absorbierenden Substrat befestigt ist, wobei die Trennbeschichtung (50, 55, 57) und der Klebstoff (40, 42, 45, 46) freiliegen, um die der Unterwäsche zugewandte Seite (20) einer Slipeinlage (10) zu bilden;
- daß die Trenneinrichtungen (50, 55, 57) in trennbarer Weise an den Befestigungseinrichtungen befestigt sind;
- daß die Slipeinlage (10) entlang einer zentralen Faltlinie, die sich in etwa in Querrichtung zur Slipeinlage (10) erstreckt und in etwa auf einer zentralen Achse (60) zwischen dem ersten und zweiten Ende der Slipeinlage (10) angeordnet ist, gefaltet wird;
- daß die Slipeinlage (10) entlang zweier weiterer Faltlinien, die symmetrisch zur zentralen Achse (60) angeordnet sind, gefaltet ist, um eine kompakte, unauffällige Slipeinlage (10) bereitzustellen, die während der Lagerung im gefalteten Zustand verbleibt;
- daß die zentrale Trenneinrichtung (55) in trennbarer Weise im Eingriff mit den äußersten Hafteinrichtungen (40 und 45) steht, die an den Längsenden der Slipeinlage (10) angeordnet sind;
- daß die sich zentral erstreckenden Trenneinrichtungen (50, 57) in trennbarer Weise im Eingriff mit den innersten Hafteinrichtungen (42, 46) stehen;
- daß die Trenneinrichtungen (50, 55, 57) eine größere Fläche als die Hafteinrichtungen (40, 42, 45, 46) aufweisen, so daß die Hafteinrichtungen (40, 42, 45, 46) und die Trenneinrichtung (50, 55, 57) einen Spielraum zum Ausgleich von leichten Faltungsfehlern oder Fehlern bei der Anordnung der Einrichtungen aufweisen, wodurch die Notwendigkeit für Trennpapier entfällt, jedoch die Slipeinlage (10) aufgefaltet und verwendet werden kann; und
- daß die Trenneinrichtungen (50, 55, 57) und die Hafteinrichtungen (40, 42, 45, 46) miteinander so in Kontakt stehen, daß die Slipeinlage (10) vor der Verwendung aufgefaltet werden kann, ohne daß die Klebeeinrichtung (40, 42, 45, 46) aneinander oder am Sperrfilm kleben, und am Schrittbereich der Unterwäsche befestigt werden kann.

7. Verfahren nach Anspruch 6, dadurch gekennzeichnet,
- daß eine Rolle eines mit Mikroprägungen versehenen Polyethylen-Sperrfilms von 38,1 µm (1,5 mil) zunächst auf eine Breite von 155,6 mm (6,125") geschlitzt und sodann einer Koronabehandlung zur Erzielung einer Oberflächenenergie von etwa 0,0038 N/cm auf der zu beschichtenden Seite unterzogen wird;
- daß Silikon auf die koronabehandelte Seite unter Verwendung einer Siebdruckvorrichtung aufgedruckt wird;
- daß ein UV-härtbares Silikon-Trennmittel hinter einem Rakelmesser im Innern eines Drucksiebs zugesetzt wird;
- daß das Silikon-Trennmaterial unter Verwendung eines UV-Härtungssystems und bei einer Maschinengeschwindigkeit von etwa 60,96 m/min gehärtet wird;
- daß Schmelzkleber-Haftstreifen (40, 42, 45, 46) auf jede der vier Zonen auf einer im Vergleich zur Gesamtheit der einzelnen Befestigungszonen kleineren Fläche aufgebracht wird;
- der gleiche Schmelzkleber auf die dem Absorptionsmittel zugewandte Seite des Sperrfilms aufgesprüht wird, der als laminierender Klebstoff verwendet wird, und daß die absorbierende Watte gegen den Laminationsklebstoff angedrückt wird;
- daß der Verbundstoff aus Sperre und Absorptionsmittel an den Enden gefältelt wird;
- daß die Produktenden zugeschnitten werden, um ein abgerundetes Lappenende zu bilden;
- daß die Slipeinlage (10) in der Mitte um die zentrale Faltlinie (60), die quer zur Slipeinlage (10) ausgerichtet ist, gefaltet wird, so daß die körperzugewandten Seiten (30) miteinander in Kontakt kommen; und
- die Querenden erneut gefaltet werden, so daß die Trenn- und Befestigungseinrichtungen (50, 55, 57) so aufeinandertreffen, daß die Hafteinrichtungen (40, 42, 45, 46) mit den Stellen (50, 55, 57) der Trennbeschichtung in Kontakt kommen, was eine leichte, beschädigungsfreie Auffaltung der Slipeinlage (10) ermöglicht und das Verwerfen eines Trennpapiers entbehrlich macht.

## Revendications

1. Article absorbant (10) ayant
- un côté exposé au corps (30) comprenant un corps absorbant, un côté exposé au vêtement (20) comprenant des moyens de voile de protection imperméables aux fluides, des extrémités transversales et des bords longitudinaux ; lesdits moyens de voile de protection étant fournis avec au moins deux moyens de fixation comprenant des moyens adhésifs autocollants (40, 42, 45, 46) et avec au moins un moyen de séparation (50, 55, 57),
- une ligne de pliage centrale s'étendant approximativement transversalement à travers ledit article absorbant (10) et étant disposée approximativement sur un axe central (60) entre les première et deuxième extrémités dudit article absorbant (10) ;
ledit article absorbant (10) étant plié le long de ladite ligne de pliage centrale (60) de sorte que les moyens adhésifs (40, 42, 45, 46) soient en contact avec les moyens de séparation (50, 55, 57) ;
lesdits moyens de séparation (50, 55, 57) étant fixés de façon détachable aux moyens de fixation ;
lesdits moyens adhésifs (40, 42, 45, 46) sont complètement couverts par lesdits moyens de séparation (50, 55, 57) ;
caractérisé en ce que
- ledit article absorbant est un protège-slip (10) ;
- lesdits moyens adhésifs (40, 42, 45, 46) sont appliqués au côté exposé au sous-vêtement (20) en traçant des zones de fixation, et lesdits moyens adhésifs (40, 42, 45, 46) sont disposés sur au moins une partie des zones de fixation pour fixer ledit protège-slip (10) en contact direct rapproché entre la surface périnéale et le sous-vêtement de l'utilisatrice ;
- un premier (55) desdits moyens de séparation (50, 55, 57) s'étend au centre symétriquement autour de l'axe central (60) du protège-slip (10), et un deuxième (50, 57) desdits moyens de séparation (50, 55, 57) est disposé en plus symétriquement autour de l'axe central (60) ;
- deux lignes de pliage supplémentaires sont réalisées de façon symétrique autour de l'axe central (60) de façon à fournir un protège-slip discret et compact (10) ;
lesdits moyens de séparation centraux (55) s'engagent de façon détachable avec les moyens adhésifs les plus à l'extérieur (40 et 45) qui sont disposés aux extrémités longitudinales du protège-slip (10) ;
lesdits moyens de séparation s'étendant au centre (50, 57) s'engagent de façon détachable avec les moyens adhésifs les plus à l'intérieur (42, 46) ;
lesdits moyens de séparation (50, 55, 57) sont plus grands en surface que les moyens adhésifs (40, 42, 45, 46) de sorte que les moyens adhésifs (40, 42, 45, 46) et les moyens de séparation (50, 55, 57) disposent d'une tolérance pour de légères erreurs de pliage ou des erreurs de placement de moyens éliminant la nécessité d'un papier de séparation, mais permettant au protège-slip (10) d'être déplié et utilisé.

2. Article absorbant selon la revendication 1, caractérisé en ce que
- lesdits moyens adhésifs sont des bandes adhésives (40, 42, 45, 46) ;
- un espace est fourni entre les deux bandes adhésives les plus à l'intérieur (42, 46) et un espace est fourni entre les bandes adhésives les plus à l'extérieur (40, 45) ;
- le composé de protection et d'absorbant est serti sur les extrémités qui sont coupées pour fournir une extrémité de languette ;
- les extrémités transversales sont pliées encore une fois de sorte que les moyens de fixation et de séparation (40, 42, 45, 46; 50, 55, 57) se rencontrent ;
- le protège-slip (10) est plié en deux le long de la ligne de pliage centrale (60) de sorte que les côtés exposés au corps (30) entrent en contact mutuel ;
- les extrémités transversales du protège-slip (10) sont pliées encore une fois de sorte que les moyens de séparation et de fixation se rencontrent, de sorte que les moyens adhésifs (40, 42, 45, 46) entrent en contact avec les emplacements (50, 55, 57) de pellicule de séparation, permettant au protège-slip (10) d'être déplié aisément sans dommages.

3. Article absorbant selon les revendications 1 ou 2, caractérisé par
- un voile de protection de 38,1 micromètres (1,5 mil) ayant une largeur d'environ 155,6 mm (6,125") ;
- ledit voile de protection ayant subi un traitement par effet corona pour avoir une énergie superficielle d'environ 0,0038 N/cm (38 dynes/cm) sur le côté à enduire ;
- un agent de séparation silicone de type polymérisable par radiation ultraviolette (UV) étant imprimé sur côté traité par effet corona du voile de protection et polymérisé ;
- lesdites bandes adhésives (40, 42, 45, 46) ayant une largeur d'environ 9,53 mm (3/8 pouce) et une longueur d'environ 40,64 mm (1,6 pouces), appliquées sur une surface plus petite que la totalité de la zone de fixation pour créer lesdits moyens de séparation (50, 55, 57), étant plus grandes que les bandes adhésives (40, 42, 45, 46) ;
- ledit espace entre les deux bandes adhésives les plus à l'intérieur (42, 46) étant d'environ 38,1 mm (1,5 pouces) ;
- ledit espace entre les bandes adhésives les plus à l'extérieur (40, 45) étant d'environ 22,225 mm (0,875 pouces).

4. Article absorbant selon la revendication 1 ou 2, caractérisé en ce que l'agent de séparation (50, 55, 57) est de la silicone.

5. Article absorbant selon la revendication 1 caractérisé en ce que le protège-slip (10) a la forme d'un rectangle.

6. Procédé de fabrication d'un article absorbant (10) selon l'une quelconque des revendications 1 à 5, caractérisé en ce que
- il fournit un voile de protection ;
- ledit voile de protection est d'abord imprimé avec une pellicule de séparation (50, 55, 57) dans une zone de séparation ;
- un adhésif (40, 42, 45, 46) est appliqué aux zones de fixation disposées sur le même côté (20) que la pellicule de séparation (50, 55, 57) ;
- un adhésif est enduit aussi sur un côté exposé à l'absorbant (30) du voile de protection pour aider à la stratification du voile sur un absorbant ;
- l'adhésif (40, 42, 45, 46) dans les zones de fixation est appliqué de façon à de ne pas occuper toute l'aire de surface des zones de fixation ;
- le côté exposé à l'absorbant (30) de la protection est attaché à un substrat absorbant, laissant la pellicule de séparation (50, 55, 57) et l'adhésif (40, 42, 45, 46) exposés pour former le côté exposé au sous-vêtement (20) d'un protège-slip (10) ;
- lesdits moyens de séparation (50, 55, 57) sont fixés de façon détachable aux moyens de fixation ;
- ledit protège-slip (10) est plié le long d'une ligne de pliage centrale s'étendant approximativement transversalement à travers ledit protège-slip (10) et est disposé approximativement sur un axe central (60) entre les première et deuxième extrémités dudit protège-slip (10) ;
- ledit protège-slip (10) est plié le long de deux lignes de pliage supplémentaires réalisées de façon symétrique autour de l'axe central (60) de façon à fournir un protège-slip discret et compact (10) restant en position pliée pendant le stockage ;
lesdits moyens de séparation centraux (55) s'engagent de façon détachable avec les moyens adhésifs les plus à l'extérieur (40 et 45) qui sont disposés aux extrémités longitudinales du protège-slip (10) ;
- lesdits moyens de séparation s'étendent au centre (50, 57) s'engagent de façon détachable avec lesdits moyens adhésifs les plus à l'intérieur (42, 46) ;
- lesdits moyens de séparation (50, 55, 57) sont plus grands en surface que les moyens adhésifs (40, 42, 45, 46) de sorte que les moyens adhésifs (40, 42, 45, 46) et les moyens de séparation (50, 55, 57) disposent d'une tolérance pour de légères erreurs de pliage ou des erreurs de placement des moyens éliminant la nécessité d'un papier de séparation, mais permettant au protège-slip (10) d'être déplié et utilisé ;
- des moyens de séparation (50, 55, 57) et des moyens adhésifs (40, 42, 45, 46) sont en contact mutuel de sorte que le protège-slip (10) peut être déplié avant utilisation, sans que les moyens adhésifs (40, 42, 45, 46) collent tous seuls ou au voile de protection, et peut être fixé à la partie d'entrejambe d'un sous-vêtement.

7. Procédé selon la revendication 6, caractérisé en ce que
- un rouleau de 38,1 micromètres (1,5 mil) de voile de protection en polyéthylène micro-gaufré est d'abord fendu sur une largeur de 155,6 mm (6,125") et ensuite traite par effet Corona de façon à obtenir une énergie de surface d'environ 0,0038 N/cm sur le côté à enduire ;
- de la silicone est imprimée sur le côté traité par effet corona en utilisant un appareil de sérigraphie ;
- un agent de séparation silicone de type polymérisable par radiation ultraviolette est ajouté derrière un couteau nettoyeur à l'intérieur d'un écran de sérigraphie ;
- la matière de séparation silicone est polymérisée en utilisant un système de polymérisation par UV et une vitesse de machine d'environ 60,96 m/min ;
- des bandes de colle à fusion (40, 42, 45, 46) sont appliquées dans chacune de quatre zones sur une surface inférieure à la totalité de chacune des zones de fixation ;
- la même colle à fusion est vaporisée sur le côté exposé à l'absorbant du voile de projection à utiliser comme adhésif de stratification, et la batte absorbante est placée contre l'adhésif de stratification ;
- le composé de protection et d'absorbant est serti sur les extrémités ;
- les extrémités du produit sont coupées pour fournir une extrémité de languette arrondie ;
- le protège-slip (10) est plié en deux le long de la ligne de pliage centrale (60) alignée transversalement à travers le protège-slip (10) de sorte que les côtés exposés au corps (30) entrent en contact mutuel ;
- les extrémités transversales sont pliées encore une fois de sorte que les moyens de séparation et de fixation (50, 55, 57) se rencontrent, de sorte que les moyens adhésifs (40, 42, 45, 46) entrent en contact avec les emplacements (50, 55, 57) de pellicule de séparation, permettant au protège-slip (10) d'être déplié aisément sans dommages ni nécessité d'éliminer un papier de séparation.
